# FASCICULE DE BREVET EUROPEEN

(11) **EP 3 201 374 B1**
(45) Date de publication et mention de la délivrance du brevet: **06.11.2019**
(21) Numéro de dépôt: 15787256.5
(22) Date de dépôt: 29.09.2015
(51) Int. Cl.: C25B 15/02, C25B 1/02, C25B 9/08

(54) **PROCÉDÉ ET DISPOSITIF DE RÉGULATION DE L'ACTIVITÉ D'UN SYSTÈME BIOÉLECTROCHIMIQUE COMPORTANT À LA FOIS UNE BIOANODE ET UNE BIOCATHODE**
VERFAHREN UND VORRICHTUNG ZUR STEUERUNG DER AKTIVITÄT EINES BIOELEKTROCHEMISCHEN SYSTEMS MIT EINER BIOANODE UND BIOKATHODE
METHOD AND DEVICE FOR CONTROLLING THE ACTIVITY OF A BIOELECTROCHEMICAL SYSTEM COMPRISING BOTH A BIOANODE AND A BIOCATHODE

(30) Priorité: 30.09.2014 FR 1459281
(43) Date de publication de la demande: 09.08.2017
(73) Titulaire: Institut National de Recherche en Sciences et Technologies pour l'Environnement et l'Agriculture (IRSTEA), 92160 Antony (FR)
(72) Inventeur: BOUCHEZ, Théodore, 91630 Villemoisson (FR); BRIDIER, Arnaud, 35300 Fougeres (FR); LE QUÉMÉNER, Elie, 11100 Narbonne (FR)
(74) Mandataire: Le Cloirec, Claudine
(86) Numéro de dépôt international: PCT/FR2015/052585
(87) Numéro de publication internationale: WO 2016/051064

(56) Documents cités:
- WO-A1-2009/008709
- MARIANNA VILLANO ET AL: "Electrochemically assisted methane production in a biofilm reactor", JOURNAL OF POWER SOURCES, ELSEVIER SA, CH, vol. 196, no. 22, 5 juillet 2011 (2011-07-05), pages 9467-9472, XP028283674, ISSN: 0378-7753, DOI: 10.1016/J.JPOWSOUR.2011.07.016 [extrait le 2011-07-12]
- ROZENDAL R A ET AL: "Principle and perspectives of hydrogen production through biocatalyzed electrolysis", INTERNATIONAL JOURNAL OF HYDROGEN ENERGY, ELSEVIER SCIENCE PUBLISHERS B.V., BARKING, GB, vol. 31, no. 12, 1 septembre 2006 (2006-09-01), pages 1632-1640, XP024899921, ISSN: 0360-3199, DOI: 10.1016/J.IJHYDENE.2005.12.006 [extrait le 2006-09-01]
- DATABASE WPI Week 201356 Thomson Scientific, London, GB; AN 2013-H82982 XP002740410, & CN 102 925 492 A (CHENGDU BIOLOGY RES INST CHINESE ACAD O) 13 février 2013 (2013-02-13)

## Description

La présente invention concerne le domaine électrochimique, et plus particulièrement les procédés électrochimiques mettant en oeuvre des dispositifs bioélectrochimiques c'est-à-dire des dispositifs électrochimiques dont l'une au moins des électrodes est au contact de microorganismes.

Les dispositifs bioélectrochimiques sont récents. L'étude des anodes biologiques dans le cadre des biopiles a débuté au cours des années 2000 dans des dispositifs expérimentaux pour lesquels la cathode était le plus souvent abiotique. Parfois, des cathodes à air qui pouvaient éventuellement être biologiques ont toutefois été utilisées.

Les dispositifs bioélectrochimiques font l'objet d'une attention accrue ces dernières années en vue d'étudier la valorisation et la bioconversion de la matière organique notamment issue de déchets organiques dans des électrolyseurs microbiens. Ces dispositifs bioélectrochimiques nécessitent le couplage d'une anode biologique (appelée aussi bioanode) à une cathode biologique (appelée aussi biocathode) et requièrent une bonne coordination cinétique des activités biologiques à l'anode et à la cathode. Souvent, ces activités ne sont pas bien synchronisées. Ainsi, l'activité de l'une des bioélectrodes prend en général le pas et peut entraîner, dans le circuit, des potentiels ou des courants néfastes au bon fonctionnement de l'autre bioélectrode.

Jusqu'à présent l'étude des biocathodes pour l'électrosynthèse n'a pratiquement jamais été réalisée dans un système comportant également une bioanode. Pourtant, pour les futures applications industrielles de bioraffinage des déchets, l'utilisation conjointe d'une bioanode et d'une biocathode est nécessaire.

Un premier but de l'invention est donc de palier les inconvénients des procédés existants en proposant de réguler les variations d'activités respectives des bioélectrodes, c'est-à-dire la coordination des activités microbiennes à la fois sur la bioanode et sur la biocathode d'un dispositif électrochimique dont les compartiments anodique et cathodique renferment des microorganismes.

A cet effet la présente invention propose un procédé de régulation de l'activité d'un dispositif électrochimique comportant une anode et une cathode plongées dans un électrolyte respectivement placé dans un compartiment anodique et un compartiment cathodique séparés par au moins une membrane ou reliés l'un à l'autre par un pont salin, le dispositif comportant éventuellement une électrode de référence, une différence de potentiel étant appliquée entre l'anode et la cathode, ou entre l'anode et l'électrode de référence, caractérisé en ce que l'électrolyte du compartiment anodique, ainsi que l'électrolyte du compartiment cathodique, contiennent des microorganismes en suspension ou sous forme de biofilm(s), les électrodes étant respectivement dénommées bioanode et biocathode, et en ce que le fonctionnement du dispositif est régi par une double régulation :
- une première régulation, dite régulation prioritaire, de la différence de potentiel entre la bioanode et la biocathode, ou entre la bioanode et l'électrode de référence, entre une valeur limite minimale permettant le développement d'un biofilm électroactif à la bioanode et une valeur limite maximale inférieure au potentiel d'oxydation dudit biofilm, et
- une seconde régulation, dite régulation secondaire, lorsque la première régulation est mise en place, optimisant le rendement faradique de la biocathode.

Classiquement, dans les expériences d'électrosynthèse microbienne, les réactions de réduction ayant lieu sur les biocathodes, les dispositifs électrochimiques de l'art antérieur s'appuient sur une simple régulation du courant ou du potentiel à la cathode. Lorsqu'on est en présence d'une bioanode, ce type de régulation peut entraîner une augmentation forte du potentiel à l'anode qui conduit à l'inactivation de la biomasse électroactive par une oxydation trop importante au contact de cette électrode.

Or, de façon surprenante et inattendue, les inventeurs ont constaté qu'il est préférable d'employer, au démarrage, une stratégie de régulation du potentiel à l'anode. Une fois que l'activité microbienne d'oxydation à l'anode permet d'atteindre un niveau de courant dans le circuit jugé suffisant, il devient nécessaire de réguler la densité de courant et/ou le potentiel à la biocathode pour permettre une activité biologique adéquate à la cathode (régulation du courant ou du potentiel sur les plages compatibles avec l'activité de l'anode). Le procédé selon l'invention prend donc en compte en premier lieu l'évolution de l'activité biologique à l'anode, par la régulation prioritaire du potentiel à la bioanode (loi prioritaire).

Dans l'ensemble du texte, les expressions efficacité coulombique, rendement coulombique, efficacité faradique et rendement faradique sont équivalentes et ont la même signification sur le plan électrochimique.

La densité de courant (en A/m2) à la bioanode ou à la biocathode correspond à l'intensité par unité de surface de l'électrode dont il est question.

Par "bioanode" et "biocathode", on entend, ici, des électrodes plongées dans un électrolyte au contact de microorganismes et d'un électrolyte. Ces microorganismes peuvent s'organiser en biofilms directement au contact des électrodes, des parois du réacteur et/ou être en suspension dans l'électrolyte. Dans l'ensemble du texte on utilisera indifféremment les termes d'anode ou de bioanode, et de cathode ou de biocathode.

De manière avantageuse, tout en maintenant la régulation du potentiel à la bioanode, l'optimisation de l'activité de la biocathode en vue de la production d'espèces chimiques particulières est asservie à des paramètres physico-chimiques mesurés au niveau du compartiment cathodique, telles que la concentration d'une ou plusieurs espèces chimiques dans l'électrolyte ou dans ciel gazeux environnant la biocathode, ou au débit de production de gaz à la biocathode.

L'espèce chimique mesurée au niveau du compartiment cathodique, est par exemple le dihydrogène (résultant d'un phénomène abiotique d'électrolyse de l'eau et signifiant que l'intensité doit être réduite pour améliorer le fonctionnement de la biocathode) ou le méthane (qui signe le développement d'une électro-méthanogenèse à la biocathode).

Selon un mode de réalisation avantageux de l'invention, tout en maintenant la première régulation de différence de potentiel gérant l'activité biologique au niveau de la bioanode, la seconde régulation régit l'optimisation de la densité de courant à la biocathode en vue de la production d'espèces chimiques particulières au niveau de la biocathode.

Cette optimisation de densité de courant à la biocathode peut être réalisée :
- selon une première variante : par le biais d'un dispositif électronique permettant de fixer directement l'intensité à la biocathode, et/ou par le biais d'un générateur de tension ou d'un potentiostat, permettant de faire varier, de préférence avec une précision de quelques millivolts, le potentiel de la bioanode, de la biocathode et/ou la différence de potentiel entre bioanode et biocathode, ou
- selon une seconde variante : par des variations du rapport de la surface active de la bioanode à la surface active de la biocathode.

Dans cette seconde variante, les variations du rapport de la surface active de la bioanode à la surface active de la biocathode peuvent être réalisées :
- soit par des variations de la surface immergée de la bioanode et/ou de la biocathode, ces variations de la surface immergée de la bioanode et/ou de la biocathode pouvant être obtenues par des variations du niveau de l'électrolyte dans le compartiment anodique et/ou dans le compartiment cathodique, ou encore obtenues par des déplacements de la bioanode et/ou de la biocathode dans l'électrolyte ;
- soit par la modification du nombre de bioanodes dans le compartiment anodique et/ou la modification du nombre de biocathodes dans le compartiment cathodique : c'est-à-dire l'introduction d'une ou plusieurs nouvelles bioanodes (respectivement biocathodes) dans le compartiment anodique (respectivement cathodique), ou le retrait d'une ou plusieurs bioanodes et/ou biocathodes, pour le cas où ce(s) compartiment(s) en comprend(nent) déjà plusieurs.

Dans les cas où les compartiments anodique et cathodique comprennent plusieurs électrodes (respectivement bioanodes ou biocathodes), ces électrodes sont bien entendues toutes connectées au même circuit électrique du dispositif électrochimique.

Selon un autre mode de réalisation, l'activité de la biocathode peut être régulée par des paramètres physico-chimiques au niveau du compartiment anodique. Plus particulièrement, tout en maintenant la régulation du potentiel à la bioanode, l'optimisation de l'activité de la biocathode en vue de la production d'espèces chimiques particulières est alors réalisée par la régulation de paramètres chimiques au niveau du compartiment anodique, telles que la concentration d'une ou plusieurs espèces chimiques dans l'électrolyte ou dans ciel gazeux environnant la bioanode.

Cette espèce chimique est avantageusement une molécule non fermentescible, telle qu'un acide organique ou son sel, de préférence choisi parmi l'acétate, le lactate ou le propionate.

A titre d'exemple de régulation prioritaire, lorsque la bioanode, telle qu'une électrode en carbone, est plongée dans un électrolyte aqueux à pH d'environ 7 la valeur maximale de la différence de potentiel entre la bioanode et une électrode normale à hydrogène, dite électrode de référence, est telle que le potentiel de la bioanode est inférieur ou égal à 1 V par rapport à ladite électrode de référence, de préférence inférieur à 0,5 V par rapport à ladite électrode de référence, de manière à éviter l'électrolyse de l'eau.

La présente invention concerne également le dispositif électrochimique pour la réalisation du procédé décrit ci-dessus, permettant l'adaptation des courants et/ou potentiels à la cathode en fonction de l'évolution de l'activité biologique à l'anode.

Plus particulièrement le dispositif électrochimique selon l'invention comprenant :
- une anode et une cathode plongées dans un électrolyte respectivement placé dans un compartiment anodique et un compartiment cathodique séparés par au moins une membrane ou reliés l'un à l'autre par un pont salin, l'électrolyte du compartiment anodique contenant des microorganismes, ainsi que l'électrolyte du compartiment cathodique, les électrodes étant respectivement dénommées bioanode et biocathode,
- éventuellement une électrode de référence,
- des moyens permettant d'appliquer une différence de potentiel entre la bioanode et la biocathode, ou entre la bioanode et l'électrode de référence, et des moyens de régulation de cette différence de potentiel,
est caractérisé en ce qu'il comprend des moyens permettant d'optimiser le rendement faradique de la biocathode, l'optimisation étant asservie à des paramètres physico-chimiques mesurés au niveau du compartiment cathodique et/ou au niveau du compartiment anodique.

La membrane peut être une membrane échangeuse d'ions : échangeuse de cations, échangeuse d'anions, échangeuse de protons, ou en variante être une membrane d'osmose.

Selon l'invention ce dispositif électrochimique comprend également des capteurs ou sondes disposés dans l'électrolyte et/ou dans le ciel gazeux environnant respectivement la bioanode ou la biocathode, lesdits capteurs ou sondes mesurant des paramètres physico-chimiques au niveau du compartiment anodique et/ou du compartiment cathodique, telles que la concentration d'une ou plusieurs espèces chimiques.

Le dispositif électrochimique selon l'invention peut également comprendre des moyens permettant de faire varier le niveau de l'électrolyte dans au moins un des compartiments (par exemple au moyen d'une pompe connectée à une entrée et une sortie du compartiment correspondant) et/ou permettant le déplacement de la bioanode et/ou de la biocathode dans l'électrolyte.

L'invention sera bien comprise à la lecture de la description suivante d'exemples de réalisation, en référence aux dessins annexés dans lesquels :
La figure 1 est un schéma en coupe d'un mode de réalisation d'un dispositif électrochimique selon l'invention ;
La figure 2 est un schéma en coupe d'un autre mode de réalisation d'un dispositif électrochimique selon l'invention;
La figure 3 montre les courbes de densité de courant (figure 3A) et de potentiel (figure 3B) de l'exemple 1 ;
La figure 4 schématise, pour l'exemple 1, le bilan des électrons au niveau de la biocathode de l'exemple 1 avec les quantités cumulées d'acides gras volatils (AGVs), de méthane (CH₄) et de dihydrogène (H₂) (figure 4A) et les acides gras volatils mesurées au niveau du compartiment cathodique ;
La figure 5 montre l'intensité dans le dispositif électrochimique selon l'exemple 2 en fonction du temps ;
La figure 6 montre l'intensité dans le dispositif électrochimique selon l'exemple 3 en fonction du temps, et les déplacements de la bioanode dans l'électrolyte ;
La figure 7 montre l'intensité dans deux dispositifs électrochimiques selon l'exemple 4 en fonction du temps ;
La figure 8 montre le potentiel dans deux dispositifs électrochimiques selon l'exemple 4 en fonction du temps ;
La figure 9 est un schéma général d'un dispositif électrochimique selon l'invention comportant différents capteurs physico-chimiques ;
La figure 10 montre des courbes de variation d'intensité au cours du temps dans trois dispositifs selon l'invention fonctionnant à des différences de potentiel variées entre l'anode et la cathode ;
La figure 11 est un schéma général d'une variante de dispositif électrochimique selon l'invention comportant une alimentation en continu de l'électrolyte dans le compartiment anodique ;
La figure 12 montre la corrélation entre l'intensité du courant dans le circuit du dispositif de la figure 11 et la charge journalière en DCO (demande chimique en oxygène) dans l'alimentation en électrolyte du compartiment anodique.

### EXEMPLES

### Exemple 1

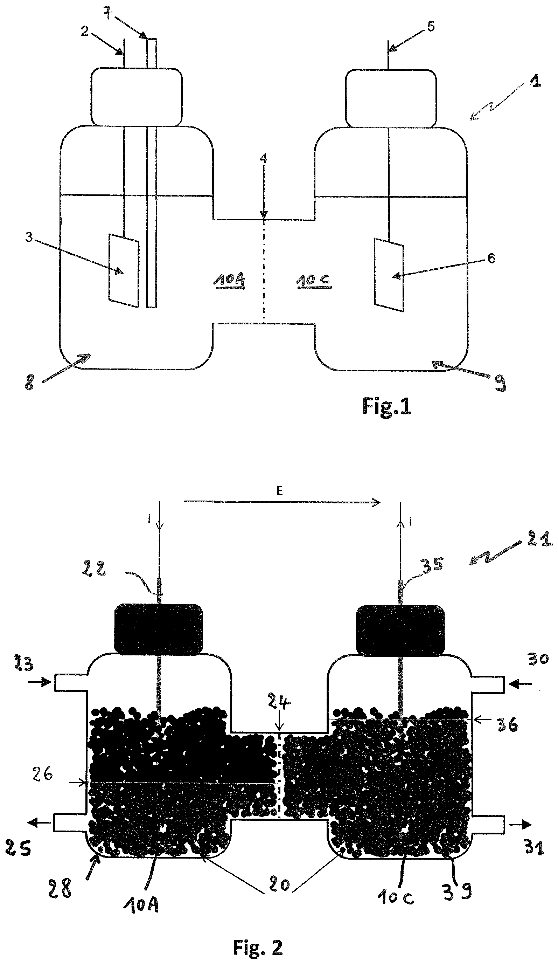

Le dispositif électrochimique 1, présenté à la figure 1, est un électrolyseur à double compartiment comportant une bioanode 3 et une biocathode 6. Les deux compartiments anodique 8 et cathodique 9 sont constitués de récipients en verre de 1,5 L de volume total séparés par une membrane 4 échangeuse de cations (MEC, Fumasep® FKE, Germany). L'électrolyte 10A, 10C utilisé correspond au milieu Biochemical Methane Potential (BMP) (selon la norme NF EN ISO 11734) tamponné avec 8 g/l de carbonates. Le substrat utilisé est l'acide acétique à 600 mg/l. Le matériau de base de la bioanode est un morceau de 4 cm ^{∗} 4 cm de tissu de carbone (Paxitech®, France) il est connecté au circuit électrique par un fil de platine 2. Le matériau de la biocathode est une plaque d'acier inoxydable (Outokumpu®, 254 SMO) de 4 cm ^{∗} 4 cm connectée au circuit électrique par une tige d'acier 5.

La bioanode a été "pré-cultivée" sur acide acétique dans des boues biologiques ce qui a permis d'obtenir à sa surface un biofilm contenant des bactéries électroactives sur le tissu de carbone. Le compartiment cathodique 9 peut recevoir ou non l'inoculation d'une culture de bactéries acétogènes préparée à partir d'un consortium microbien de traitement anaérobie de déchets (biocathode).

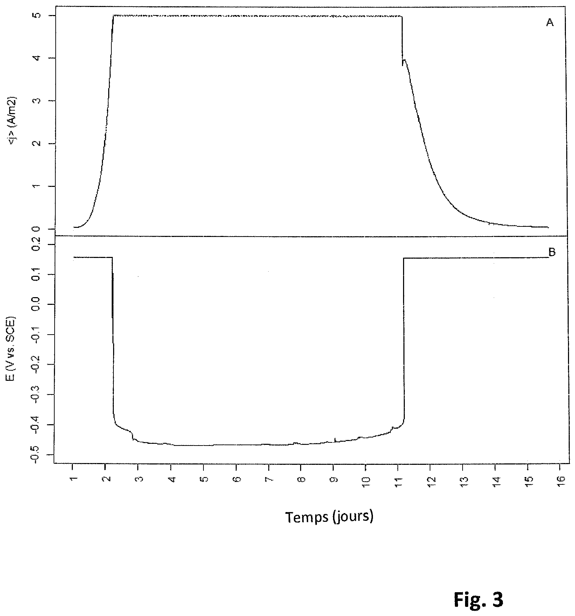

L'anode 3 est, dans un premier temps, polarisée à +0,158 V par rapport à une électrode de référence 7 au calomel saturé (notée SCE pour Saturated Calomel Electrode) au moyen d'un potentiostat (BioLogic®, France, VMP3, logiciel EC-Lab) (loi prioritaire). Lorsque la densité de courant atteint 5 A/m², le potentiostat est programmé pour limiter la densité de courant à cette valeur. Ceci permet de réguler l'activité de la cathode (loi secondaire). Enfin si le potentiel à l'anode dépasse +0,2V versus SCE, le potentiostat est alors réglé pour rebasculer en contrôle potentiel et le maintenir à cette valeur pour préserver l'activité biologique de la bioanode (loi prioritaire). Cette régulation est illustrée par la figure 3 sur les 16 premiers jours de l'expérience.

La figure 3 montre les courbes de la densité de courant (figure 3A) et du potentiel de la bioanode mesuré par rapport à l'électrode de référence 7 au calomel saturé (figure 3B) au cours des 16 premiers jours de l'expérience. En début d'expérience le courant augmente rapidement avec le développement du biofilm électroactif à l'anode. Le courant est ensuite régulé à 5 A/m² pour optimiser le rendement de production d'acides gras volatils (AGVs) à la cathode (loi secondaire). Dans la dernière phase le courant décroit avec l'épuisement du substrat pendant que la loi prioritaire impose à nouveau le potentiel à l'anode 3.

Tout au long de l'expérience sont prélevés des échantillons dans lesquels sont mesurées les concentrations en acides gras volatils et en lactate par chromatographie ionique (DIONEX DX 120, colonne IONPAC® ICE-AS1 (9x250 mm)). Les éluants utilisés sont l'acide heptafluorobutyrique (0,4 mmol/l) et l'hydroxyde de tetrabutylammonium (TBAOH, 5 mmol/l). Les concentrations en formiate, acétate, lactate, propionate, butyrate et valérate sont ainsi mesurées dans une gamme allant de 10 mg/l à 500 mg/l. Les compositions des ciels gazeux des compartiments anodiques et cathodiques sont mesurées par chromatographie en phase gazeuse (Varian CP 4900). Les trois colonnes de l'instrument permettent de mesurer les proportions des gaz suivants : O₂, N₂, CH₄, CO₂, H₂, H₂S et NO₂.

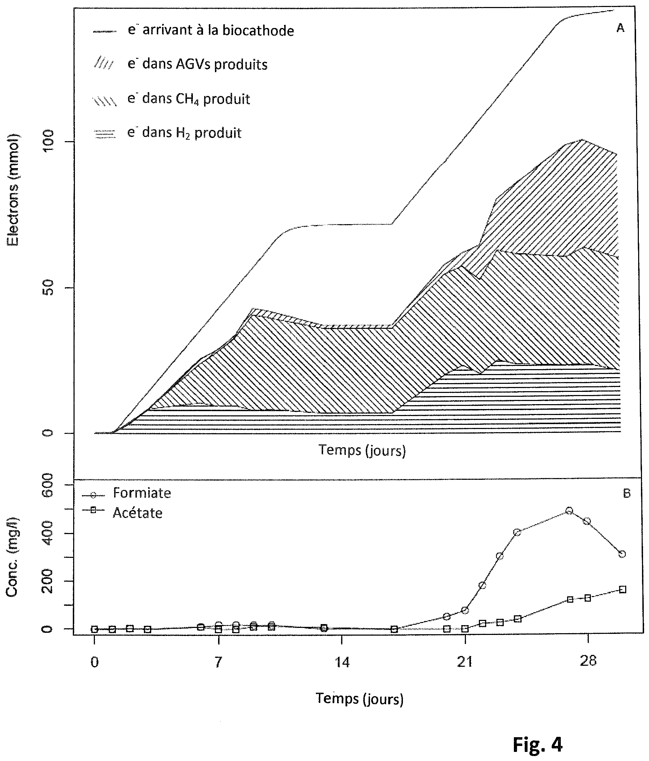

L'expérience a été menée pendant 30 jours, avec deux injections de substrat dans le compartiment anodique 8 (600 mg/l d'acide acétique) aux jours 0 et 15. Trois pilotes ont été lancés en parallèle dans les mêmes conditions avec inoculation à la cathode (biocathode) et trois autres pilotes témoins ont été lancés sans inoculation à la cathode. Les comportements des trois pilotes avec inoculation se sont révélés très similaires tout au long de l'expérience, l'un d'eux a été arrêté au jour 15 pour permettre l'observation des électrodes par microscopie. Le bilan de la production de la biocathode de l'un de ces pilotes est présenté à la figure 4 (parties A et B).

La figure 4A montre le bilan des électrons pour une biocathode 6 avec inoculation. La courbe supérieure indique la quantité d'électrons arrivant à la biocathode. Les quantités cumulées d'acides gras volatils (AGVs), de méthane (CH₄) et d'hydrogène (H₂), mesurées en équivalents électrons, sont indiquées par les surfaces hachurées. La figure 4B montre le détail des concentrations en acides gras volatils mesurées dans le compartiment cathodique 9. Le formiate et l'acétate sont les principaux acides produits. Les concentrations mesurées pour les autres acides (lactate, propionate, butyrate et valérate) sont nulles et ne sont donc pas représentées sur cette figure.

Durant la première phase de l'expérience (jours 1 à 15), l'hydrogène (H₂) et le méthane (CH₄) sont principalement produits au niveau des compartiments cathodiques ayant reçu une inoculation tandis que les pilotes sans inoculation dans le compartiment cathodique produisent uniquement de l'hydrogène. Le rendement faradique à la biocathode est alors de 60% avec 53% des électrons utilisés pour produire du méthane (figure 4A).

Au jour 15, la deuxième injection de substrat est réalisée dans le compartiment anodique 8 et 10 mmol/l de 2-bromo-éthane sulfonate (BES) sont injectés dans le compartiment cathodique 9 pour inhiber la méthanogenèse. Durant la seconde phase, les acides gras volatils tels que le formiate et l'acétate sont principalement produits, ainsi que de faibles quantités d'hydrogène et de méthane. Le rendement faradique à la cathode atteint alors 80%. 29% des électrons ont été utilisés pour produire l'acétate. Le taux maximum de production d'acétate enregistré pendant cette phase est de 11 g d'acétate/m²/jour. Par ailleurs 18%, 19% et 13% des électrons sont utilisés pour la production de formiate, d'hydrogène et de méthane respectivement (figure 4). Des traces de caprylate ont également été détectées par des analyses de chromatographie en phase gazeuse couplée à un spectromètre de masse.

Le rendement faradique à l'anode pendant l'expérience est de 85% en moyenne.

Les observations des bioanodes et biocathodes en microscopie confocale laser à balayage à 15 jours et à 30 jours révèlent une importante colonisation microbienne des deux bioélectrodes.

### Exemple 2

Optimisation de l'intensité à la biocathode par la régulation de la différence de potentiel.

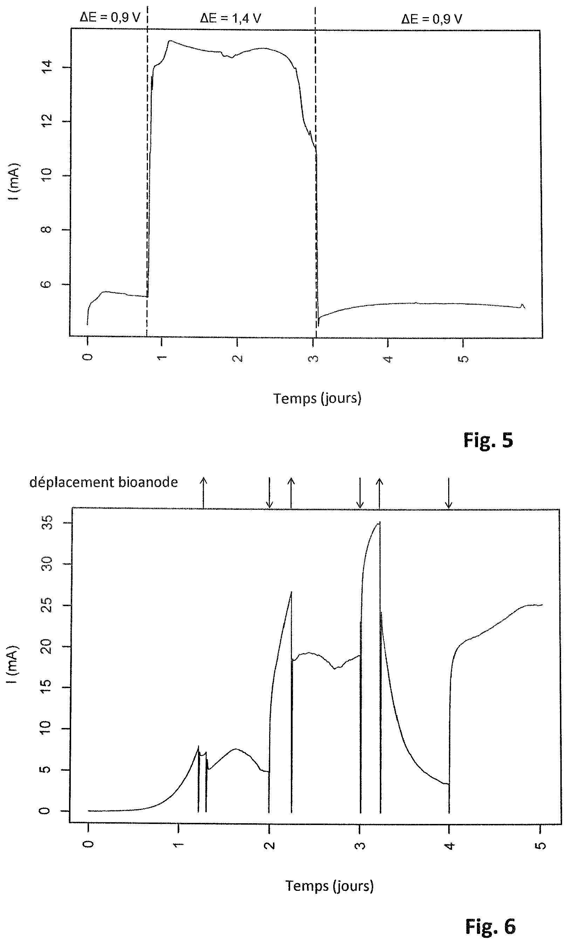

Dans cet exemple de réalisation, le réacteur bioélectrochimique est un électrolyseur à double compartiment comme celui présenté à la figure 1 et opéré sous les mêmes conditions expérimentales que l'exemple 1. Le mode de réalisation du procédé de la présente invention consiste ici à moduler la différence de potentiel entre l'anode 3 et la cathode 6 grâce à l'utilisation d'un potentiostat afin de contrôler l'intensité à la biocathode. La figure 5 présente un exemple d'intensité débitée dans le réacteur bioélectrochimique au cours du temps. Les deux différents niveaux d'intensité observés (jours 0-1 et 3-6 d'une part, jours 1-3 d'autre part) correspondent à deux différences de potentiel imposées entre la bioanode et la biocathode. La différence de potentiel imposée a été soit 0,9 V (jours 0-1 et 3-6) soit 1,4 V (jours 1-3).

Plus particulièrement, dans la première phase de l'expérience, la différence de potentiel est établie à 0,9 V entre la bioanode et la biocathode. Après environ un jour d'expérience, la différence de potentiel est augmentée à 1,4 V entre les deux électrodes. La courbe d'intensité en fonction du temps obtenue confirme une augmentation de l'intensité due à cette variation de la différence de potentiel, passant d'environ 5-6 mA à 15 mA. Après 4 jours, la différence de potentiel est rétablie à 0,9 V et est corrélée avec un retour de l'intensité à une valeur très proche de sa valeur initiale (environ 5 mA). Le contrôle de la différence de potentiel entre bioanode et biocathode permet donc de réguler l'intensité du courant au sein du réacteur bioélectrochimique et par conséquent l'activité de la biocathode.

### Exemple 3

Optimisation de l'intensité à la biocathode par déplacement de la bioanode dans l'électrolyte.

Dans cet exemple de réalisation, le réacteur bioélectrochimique est un électrolyseur à double compartiment comme celui présenté à la figure 1 et opéré sous les mêmes conditions expérimentales que l'exemple 1. L'invention consiste ici à moduler la surface de bioanode immergée dans l'électrolyte afin de faire varier l'intensité à la biocathode. La figure 6 présente un exemple d'intensité débitée dans le réacteur bioélectrochimique au cours du temps. Les déplacements de la bioanode dans le compartiment sont indiqués par les flèches. Au départ, la bioanode 3 est complètement immergée dans l'électrolyte 10A. Les flèches vers le haut indiquent que la moitié de l'électrode passe en position émergée, les flèches vers le bas indiquent que l'électrode est à nouveau immergée en totalité. On constate que les variations brusques d'intensité correspondent bien à des variations de la surface immergée de la bioanode.

En effet, dans cet exemple de réalisation, la surface immergée de la bioanode est modifiée plusieurs fois pendant l'expérience. Après un peu plus de 1 jour, la moitié de la bioanode 3 est sortie de l'électrolyte réduisant la surface immergée de moitié. On observe parallèlement à cela une baisse de l'intensité de 8 mA à 5 mA environ. Au deuxième jour, la bioanode est replongée totalement dans l'électrolyte et on note alors une hausse brutale de l'intensité. L'expérience est ainsi répétée 3 fois et permet d'observer à chaque variation de la surface immergée de la bioanode une variation corrélée de l'intensité à la biocathode. Le contrôle de la surface immergée de la bioanode permet donc de réguler l'intensité du réacteur bioélectrochimique et par conséquent l'activité de la biocathode.

### Exemple 4

Régulation de l'intensité à la biocathode en fonction de la concentration en DCO (Demande chimique en Oxygène) à l'anode.

Dans cet exemple de réalisation, le réacteur bioélectrochimique est un électrolyseur à double compartiment comme celui présenté en détail à la figure 1 et opéré sous les mêmes conditions expérimentales que l'exemple 1 à l'exception de la nature du substrat apporté à l'anode. En effet dans cette réalisation, les compartiments anodiques de deux réacteurs ont été alimentés en biodéchets, un substrat complexe de 63 g/l de DCO totale, dont la composition en acides gras volatils et en acide lactique est présentée dans le tableau 1 ci-dessous.

**Tableau 1**

| | **Conc. (g/l)** |
|---|---|
| Acide lactique | 10,52 |
| Acide formique | 0,00 |
| Acide acétique | 0,38 |
| Acide propionique | 1,28 |
| Acide butyrique | 5,32 |
| Acide valérique | 0,00 |

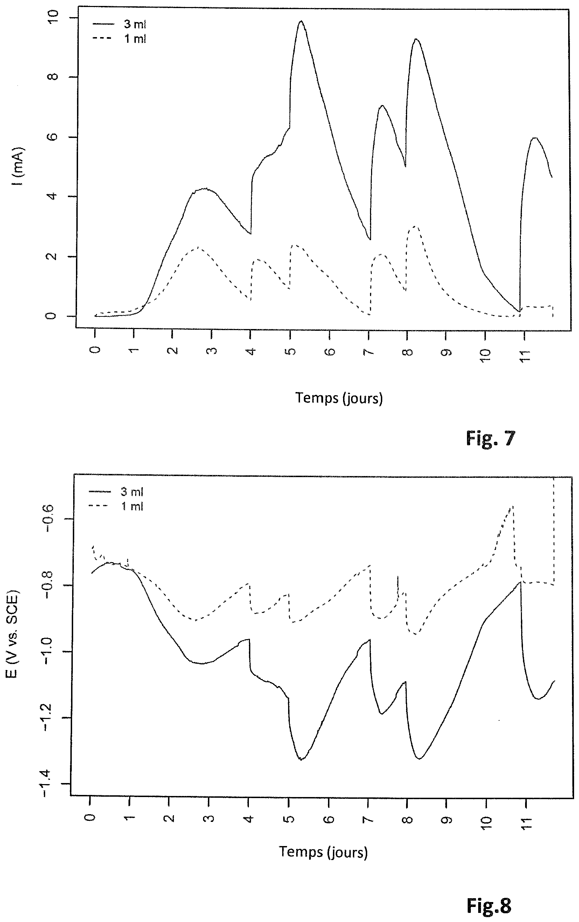

La figure 7 présente les courbes d'intensité en fonction du temps obtenues. La courbe en pointillés correspond au réacteur dont le compartiment anodique 8 a reçu 1 ml de substrat aux jours 0, 4, 5, 7, 8 et 11. La courbe pleine correspond au réacteur dont le compartiment anodique 8 a reçu 3 ml de substrat aux jours 0, 4, 5, 7, 8 et 11.

Le réacteur le moins alimenté a reçu 31,5 mg/j/l de DCO en moyenne et le second 94,5 mg/j/l de DCO en moyenne. Cette différence de concentration en substrat a permis d'obtenir une intensité différente pour chaque réacteur puisque l'intensité moyenne obtenue pour le réacteur alimenté avec 1 ml de substrat a été de 1,1 mA, tandis que l'intensité moyenne obtenue pour le réacteur alimenté avec 3 ml de substrat a été de 4,3 mA (Figure 7). La régulation de la concentration en substrat à la bioanode permet donc de réguler l'intensité du réacteur bioélectrochimique et par conséquent de réguler l'activité de la biocathode comme illustré par les potentiels mesurés aux biocathodes : voir la figure 8 qui montre les potentiels des biocathodes des deux réacteurs bioélectrochimiques au cours du temps. Ces potentiels sont mesurés par rapport à des électrodes de référence au calomel saturé (SCE). La courbe en pointillés correspond au réacteur qui a été alimenté avec 1 ml de substrat aux jours 0, 4, 5, 7, 8 et 11. La courbe pleine correspond au réacteur qui a été alimenté avec 3 ml de substrat aux jours 0, 4, 5, 7, 8 et 11.

### Exemple 5

Optimisation de l'activité de la biocathode par régulation de paramètres chimiques.

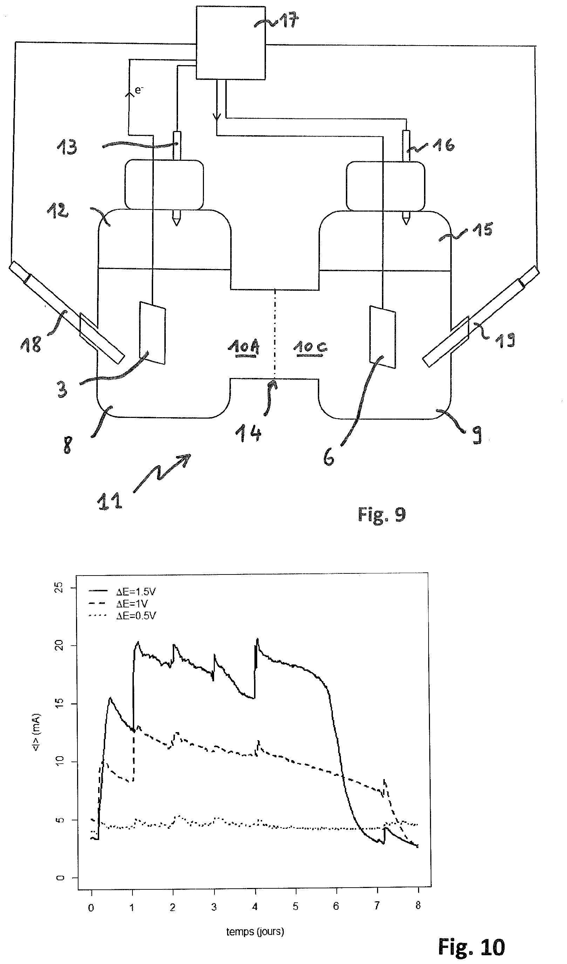

Cet exemple met en oeuvre un dispositif électrochimique comportant des capteurs physico-chimiques. Ce dispositif électrochimique 11 schématisé à la figure 9 comporte une anode 3 et une cathode 6 reliées à un dispositif électronique 17 comprenant un potentiostat. Une membrane 14, par exemple échangeuse de cations, sépare le compartiment anodique 8 du compartiment cathodique 9 renfermant l'électrolyte 10A, 10C.

Différents capteurs ou sondes peuvent être intégrés aux deux compartiments afin de suivre l'évolution des paramètres physico-chimiques du système : par exemple, un capteur de gaz 13 disposé dans le ciel gazeux 12 du compartiment anodique et une sonde 18 plongeant dans l'électrolyte 10A liquide du compartiment anodique 8. De même, un capteur 16 de gaz est relié au ciel gazeux 15 du compartiment cathodique et une sonde 19 plonge dans l'électrolyte 10C du compartiment cathodique 9.

Tout en maintenant la régulation du potentiel à la bioanode, l'optimisation de l'activité de la biocathode est réalisée par la régulation de paramètres chimiques au niveau du compartiment anodique 8, telles que la concentration d'une ou plusieurs espèces chimiques dans l'électrolyte 10A ou dans le ciel gazeux 12 environnant la bioanode, le pH, etc... La présence de différents capteurs/sondes intégrés aux deux compartiments permet de suivre en direct l'évolution des paramètres physicochimiques du système. Selon la valeur de ces paramètres, le dispositif électronique 17 peut ensuite optimiser le fonctionnement de la biocathode en jouant sur l'intensité du système, le potentiel électrique, etc...

### Exemple 6

La figure 2 schématise un autre mode de réalisation d'un dispositif électrochimique selon l'invention.

Dans cet exemple de réalisation, le dispositif électrochimique 21 est un électrolyseur à double compartiment comportant une bioanode 22 et une biocathode 35 formées de granules de graphite 20 connectées au circuit électrique extérieur par des tiges de graphite. Les compartiments anodique 28 et cathodique 39 sont séparés par une membrane 24 échangeuse de cations (MEC). Le compartiment anodique 28 comporte une entrée 23 et une sortie 25 d'électrolyte 10A, par exemple reliées à des moyens de pompage (non représentés). De même, le compartiment cathodique 39 comporte une entrée 30 et une sortie 31 d'électrolyte 10C pouvant être reliées à des moyens de pompage (non représentés) indépendants des moyens de pompage reliés au compartiment anodique.

Ce dispositif permet de moduler indépendamment le niveau 26 d'électrolyte 10 dans le compartiment anodique et le niveau 36 d'électrolyte 10C dans le compartiment cathodique, donc le volume d'électrolyte dans chacun des compartiments et ainsi à adapter le rapport des surfaces actives (c'est-à-dire immergées) des deux bioélectrodes.

Dans le cas où l'on impose le courant (I), cette modulation permet de faire varier la différence de potentiel (E) entre les deux bioélectrodes. Cela permet, dans un premier temps, lors du démarrage du système, d'avoir un potentiel à l'anode compatible avec l'établissement d'une activité biologique électroactive. Dans un second temps, cela permet de contrôler les réactions de biosynthèse à la cathode régulant son potentiel.

Dans le cas où l'on impose la différence de potentiel (E) entre les deux bioélectrodes, ce système de régulation permet de moduler le courant électrique (I), ce qui va influer directement sur les efficacités carboniques de production des différentes molécules, permettant d'optimiser les rendements de conversion des électrons en molécules d'intérêt.

### Exemple 7

Optimisation de l'intensité par la régulation de la différence de potentiel (ΔE) dans un électrolyseur à double compartiment comportant des électrodes formées de granules de graphite.

Dans cet exemple de réalisation, le dispositif électrochimique 21 est un électrolyseur à double compartiment tel que celui présenté à la figure 2 comportant une bioanode 22 et une biocathode 35 formées de granules de graphite 20 connectées au circuit électrique extérieur par des tiges de graphite. Les compartiments anodique 28 et cathodique 39 sont séparés par une membrane 24 échangeuse de cations (MEC). Le compartiment anodique 28 comporte une entrée 23 et une sortie 25 d'électrolyte 10A, par exemple reliées à des moyens de pompage (non représentés). De même, le compartiment cathodique 39 comporte une entrée 30 et une sortie 31 d'électrolyte 10C pouvant être reliées à des moyens de pompage (non représentés) indépendants des moyens de pompage reliés au compartiment anodique.

Le mode de réalisation du procédé de la présente invention consiste ici à moduler la différence de potentiel entre l'anode 22 et la cathode 35 grâce à l'utilisation d'un potentiostat afin de contrôler l'intensité. La figure 10 présente un exemple d'intensité débitée au cours du temps dans trois réacteurs bioélectrochimiques opérés dans les mêmes conditions pour trois différences de potentiel appliquées : 0,5 V (courbe en pointillés), 1 V (courbe en tirets) et 1,5 V (ligne continue). Au temps t=0 jour, le substrat (acétate à une concentration finale de 600 mg/l) est ajouté et l'intensité augmente pour les trois réacteurs. Les intensités maximales obtenues atteignent respectivement des valeurs de 5, 13 et 20 mA pour les différences de potentiel appliquées de 0,5, 1 et 1,5 V, confirmant l'augmentation de l'intensité mesurée en fonction de l'augmentation de la différence de potentiel appliquée entre la bioanode et la biocathode granulaire.
Le contrôle de la différence de potentiel entre bioanode et biocathode permet donc de réguler l'intensité du courant au sein du réacteur bioélectrochimique à double compartiment comportant des électrodes formées de granules de graphite et par conséquent l'activité de la biocathode.

### Exemple 8

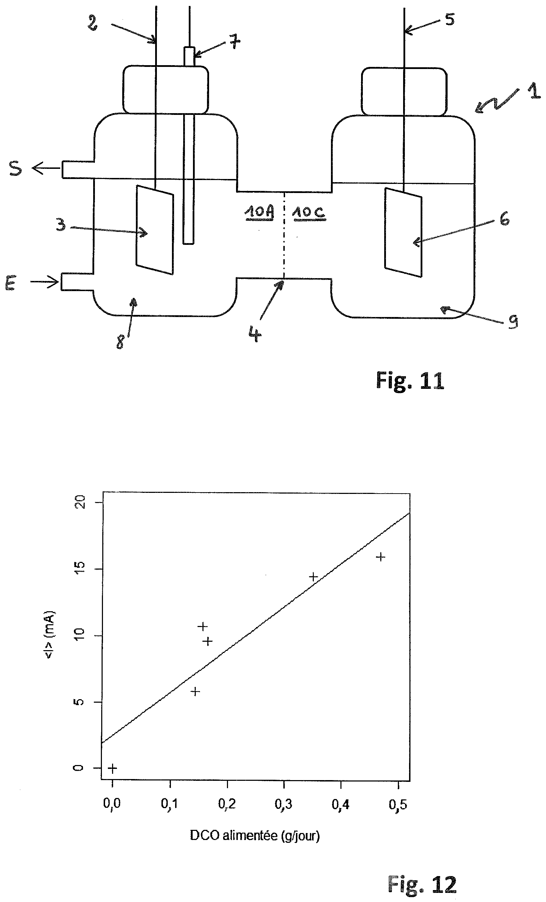

Dans cet exemple de réalisation, le dispositif électrochimique, schématisé à la figure 11, est un électrolyseur à double compartiment comportant une bioanode et une biocathode et un système d'alimentation et de soutirage de la phase liquide. Plus particulièrement, dans cet exemple, le compartiment anodique comporte une entrée E et une sortie S d'électrolyte, reliées à des moyens de pompage (non représentés) permettant une alimentation en continu de l'électrolyte dans ce compartiment.

Suivant ce mode de réalisation, le compartiment anodique d'un réacteur a été alimenté en continu en biodéchets (à partir de la composition en substrat présentée dans le tableau 1 de l'exemple 4) grâce à une pompe permettant de régler le débit entrant et à un système se surverse permettant de conserver un niveau de liquide constant au sein du compartiment anodique maintenu à un volume de 1 L.
L'impact de différentes concentrations de substrat dans l'alimentation (charge DCO : demande chimique en oxygène) sur l'intensité a été testé pour un débit moyen de 82 mL/jour sur une durée moyenne de 10 jours. Les résultats sont présentés sur la figure 12.

La corrélation constatée entre l'intensité du courant dans le circuit et le débit de DCO alimenté montre bien que la régulation du débit d'alimentation en substrat permet de réguler l'intensité du courant.

## Revendications

1. Procédé de régulation de l'activité d'un dispositif électrochimique (1) comportant une anode (3) et une cathode (6) plongées dans un électrolyte respectivement placé dans un compartiment anodique (8) et un compartiment cathodique (9) séparés par au moins une membrane (4) ou reliés l'un à l'autre par un pont salin, le dispositif comportant éventuellement une électrode de référence (7), une différence de potentiel étant appliquée entre l'anode et la cathode, ou entre l'anode et l'électrode de référence,
**caractérisé en ce que** l'électrolyte (10A) du compartiment anodique, ainsi que l'électrolyte (10C) du compartiment cathodique, contiennent des microorganismes en suspension ou sous forme de biofilm(s), les électrodes étant respectivement dénommées bioanode et biocathode, et **en ce que** le fonctionnement du dispositif est régi par une double régulation :
- une première régulation, dite régulation prioritaire, de la différence de potentiel entre la bioanode et la biocathode, ou entre la bioanode et l'électrode de référence, entre une valeur limite minimale permettant le développement d'un biofilm électroactif à la bioanode et une valeur limite maximale inférieure au potentiel d'oxydation dudit biofilm, et
- une seconde régulation, dite régulation secondaire, lorsque la première régulation est mise en place, optimisant le rendement faradique de la biocathode.

2. Procédé selon la revendication 1,
**caractérisé en ce que**, tout en maintenant la régulation du potentiel à la bioanode, l'optimisation de l'activité de la biocathode en vue de la production d'espèces chimiques particulières est asservie à des paramètres physico- chimiques mesurés au niveau du compartiment cathodique (9), telles que la concentration d'une ou plusieurs espèces chimiques dans l'électrolyte ou dans ciel gazeux environnant la biocathode, ou au débit de production de gaz à la biocathode.

3. Procédé selon la revendication 1 ou 2,
**caractérisé en ce que**, tout en maintenant la première régulation de différence de potentiel gérant l'activité biologique au niveau de la bioanode, la seconde régulation régit l'optimisation de la densité de courant à la biocathode en vue de la production d'espèces chimiques particulières au niveau de la biocathode.

4. Procédé selon la revendication 3,
**caractérisé en ce que** l'optimisation de la densité de courant à la biocathode est réalisée par le biais d'un dispositif électronique permettant de fixer directement densité de courant à la biocathode, et/ou par le biais d'un générateur de tension ou d'un potentiostat, permettant de faire varier, de préférence avec une précision de quelques millivolts, le potentiel de la bioanode, de la biocathode et/ou la différence de potentiel entre bioanode et biocathode.

5. Procédé selon la revendication 3 ou 4,
**caractérisé en ce que** l'optimisation de la densité de courant à la biocathode est réalisée par des variations du rapport de la surface active de la bioanode à la surface active de la biocathode.

6. Procédé selon la revendication 5,
**caractérisé en ce que** les variations du rapport de la surface active de la bioanode à la surface active de la biocathode sont réalisées par des variations de la surface immergée de la bioanode et/ou de la biocathode.

7. Procédé selon la revendication 6,
**caractérisé en ce que** les variations de la surface immergée de la bioanode et/ou de la biocathode sont obtenues par des variations du niveau de l'électrolyte dans le compartiment anodique et/ou dans le compartiment cathodique.

8. Procédé selon la revendication 6 ou 7,
**caractérisé en ce que** les variations de la surface active de la bioanode et/ou de la biocathode sont obtenues par des déplacements de la bioanode et/ou de la biocathode dans l'électrolyte.

9. Procédé selon l'une quelconque des revendications 5 à 8,
**caractérisé en ce que** les variations du rapport de la surface active de la bioanode à la surface active de la biocathode sont réalisées par la modification du nombre de bioanodes dans le compartiment anodique et/ou la modification du nombre de biocathodes dans le compartiment cathodique.

10. Procédé selon l'une quelconque des revendications précédentes,
**caractérisé en ce que**, tout en maintenant la régulation du potentiel à la bioanode, l'optimisation de l'activité de la biocathode en vue de la production d'espèces chimiques particulières est réalisée par la régulation de paramètres chimiques au niveau du compartiment anodique, telles que la concentration d'une ou plusieurs espèces chimiques dans l'électrolyte ou dans le ciel gazeux environnant la bioanode.

11. Procédé selon la revendication 2, **caractérisé en ce que** l'espèce chimique est le dihydrogène.

12. Procédé selon la revendication 2, **caractérisé en ce que** l'espèce chimique est le méthane.

13. Procédé selon la revendication 10, **caractérisé en ce que** l'espèce chimique est une molécule non fermentescible, telle qu'un acide organique ou son sel, de préférence choisi parmi l'acétate, le lactate ou le propionate.

14. Procédé selon l'une quelconque des revendications précédentes, **caractérisé en ce que** lorsque la bioanode, telle qu'une électrode en carbone, est plongée dans un électrolyte aqueux à pH d'environ 7 la valeur maximale de la différence de potentiel entre la bioanode et une électrode normale à hydrogène, dite électrode de référence, est telle que le potentiel de la bioanode est inférieur ou égal à 1 V par rapport à ladite électrode de référence, de préférence inférieur à 0,5 V par rapport à ladite électrode de référence, de manière à éviter l'électrolyse de l'eau.

15. Dispositif électrochimique (1) pour la réalisation du procédé selon l'une quelconque des revendications précédentes, comprenant :
- une anode (3) et une cathode (6) plongées dans un électrolyte respectivement placé dans un compartiment anodique (8;28) et un compartiment cathodique (9 ;29) séparés par au moins une membrane (4 ;14 ;24) ou reliés l'un à l'autre par un pont salin, l'électrolyte (10A) du compartiment anodique contenant des microorganismes, ainsi que l'électrolyte (10C) du compartiment cathodique, les électrodes étant respectivement dénommées bioanode et biocathode,
- éventuellement une électrode de référence (7),
- des moyens permettant d'appliquer une différence de potentiel entre la bioanode et la biocathode, ou entre la bioanode et l'électrode de référence,
- la bioanode et la biocathode étant reliées à un dispositif électronique de régulation de cette différence de potentiel,
**caractérisé en ce qu'**il comprend des moyens permettant d'optimiser le rendement faradique de la biocathode, les moyens comprenant des capteurs ou sondes disposés dans l'électrolyte (10A, 10C) et/ou dans le ciel gazeux (12, 15) environnant respectivement la bioanode (3) ou la biocathode (6), lesdits capteurs ou sondes mesurant des paramètres physicochimiques au niveau du compartiment anodique (28) et/ou du compartiment cathodique (39), telles que la concentration d'une ou plusieurs espèces chimiques.

16. Dispositif électrochimique selon la revendication 15, **caractérisé en ce que** les capteurs (13,16) ou sondes (18,19) sont reliés au dispositif électronique (17).

17. Dispositif électrochimique selon l'une quelconque des revendications 15 ou 16, **caractérisé en ce que** les compartiments anodique (28) et cathodique (39) sont séparés par une membrane (24) échangeuse de cations, le compartiment anodique comportant une entrée (23) et une sortie (25) d'électrolyte reliés à des moyens de pompage d'électrolyte, le compartiment cathodique comportant une entrée (30) et une sortie (31) d'électrolyte reliées à des moyens de pompage indépendants des moyens de pompage reliés au compartiment anodique.

18. Dispositif électrochimique selon l'une quelconque des revendications 15 à 17, **caractérisé en ce qu'**il comprend des moyens de déplacement de la bioanode (3) et/ou de la biocathode (6) dans l'électrolyte, permettant de faire varier la surface immergée de la bioanode et/ou de la biocathode.

## Patentansprüche

1. Verfahren zur Steuerung der Aktivität einer elektrochemischen Vorrichtung (1), umfassend eine Anode (3) und eine Kathode (6), die in einen Elektrolyt getaucht sind bzw. in einem anodischen Raum (8) und einem kathodischen Raum (9) angeordnet sind, die durch mindestens eine Membran getrennt (4) oder miteinander durch eine Salzbrücke verbunden sind, wobei die Vorrichtung eventuell eine Referenzelektrode (7) umfasst, wobei eine Potenzialdifferenz zwischen der Anode und der Kathode oder zwischen der Anode und der Referenzelektrode angelegt ist,
**dadurch gekennzeichnet, dass** der Elektrolyt (10A) des anodischen Raums, ebenso wie der Elektrolyt (10C) des kathodischen Raums Mikroorganismen in Suspension oder in Form eines Biofilms/von Biofilmen enthalten, wobei die Elektroden als Bioanode bzw. Biokathode bezeichnet werden, und dadurch, dass die Funktion der Vorrichtung durch eine doppelte Steuerung bestimmt wird:
- eine erste Steuerung, genannt prioritäre Steuerung, der Potenzialdifferenz zwischen der Bioanode und der Biokathode oder zwischen der Bioanode und der Referenzelektrode, zwischen einem minimalen Grenzwert, der die Entwicklung eines elektroaktiven Biofilms an der Bioanode ermöglicht und einem maximalen Grenzwert, der unter dem Oxidationspotenzial des Biofilm liegt, und
- eine zweite Steuerung, genannt sekundäre Steuerung, wenn die erste Steuerung durchgeführt wird, die die faradaische Leitsung der Biokathode optimiert.

2. Verfahren nach Anspruch 1,
**dadurch gekennzeichnet, dass**, unter Beibehaltung der Steuerung des Potenzials an der Bioanode die Optimierung der Aktivität der Biokathode angesichts der Produktion von besonderen chemischen Spezies physisch-chemischen Parametern unterworfen ist, die auf der Ebene des kathodischen Raums (9) gemessen werden, wie z. B. die Konzentration von einer oder mehreren chemischen Spezies im Elektrolyt oder in der gasförmigen Glocke, die die Biokathode umgibt, oder im Gasproduktionsdurchsatz an der Biokathode.

3. Verfahren nach Anspruch 1 oder 2,
**dadurch gekennzeichnet, dass**, unter Beibehaltung der ersten Steuerung der Potenzialdifferenz, die die biologische Aktivität auf der Ebene der Bioanode verwaltet, die zweite Steuerung die Optimierung der Stromdichte an der Biokathode angesichts der Produktion von besonderen chemischen Spezies auf der Ebene der Biokathode bestimmt.

4. Verfahren nach Anspruch 3,
**dadurch gekennzeichnet, dass** die Optimierung der Stromdichte an der Biokathode mit Hilfe einer elektronischen Vorrichtung durchgeführt wird, die ermöglicht, direkt die Stromdichte an der Biokathode zu fixieren, und/oder mit Hilfe eines Spannungsgenerators oder eines Potentiostats, der ermöglicht, vorzugsweise mit einer Präzision von einigen Millivolt, das Potenzial der Bioanode, der Biokathode und/oder die Potenzialdifferenz zwischen der Bioanode und der Biokathode zu variieren.

5. Verfahren nach Anspruch 3 oder 4,
**dadurch gekennzeichnet, dass** die Optimierung der Stromdichte an der Biokathode durch Variationen des Bezugs der aktiven Fläche der Bioanode zur aktiven Fläche der Biokathode durchgeführt wird.

6. Verfahren nach Anspruch 5,
**dadurch gekennzeichnet, dass** die Variationen des Bezugs der aktiven Fläche der Bioanode zur aktiven Fläche der Biokathode durch Variationen der eingetauchten Fläche der Bioanode und/oder der Biokathode durchgeführt werden.

7. Verfahren nach Anspruch 6,
**dadurch gekennzeichnet, dass** die Variationen der eingetauchten Fläche der Bioanode und/oder der Biokathode durch Variationen des Niveaus des Elektrolyten im anodischen Raum und/oder im kathodischen Raum erhalten werden.

8. Verfahren nach Anspruch 6 oder 7,
**dadurch gekennzeichnet, dass** die Variationen der aktiven Fläche der Bioanode und/oder der Biokathode durch Verschiebungen der Bioanode und/oder der Biokathode im Elektrolyt erhalten werden.

9. Verfahren nach einem der Ansprüche 5 bis 8,
**dadurch gekennzeichnet, dass** die Variationen des Bezugs der aktiven Fläche der Bioanode zur aktiven Fläche der Biokathode durch die Änderung de Anzahl der Bioanoden im anodischen Raum und/oder die Änderung der Anzahl der Biokathoden im kathodischen Raum durchgeführt werden.

10. Verfahren nach einem der vorhergehenden Ansprüche,
**dadurch gekennzeichnet, dass**, unter Beibehaltung der Steuerung des Potenzials an der Bioanode die Optimierung der Aktivität der Biokathode angesichts der Produktion von besonderen chemischen Spezies durch die Steuerung von chemischen Parametern auf der Ebene des anodischen Raums wie z. B. der Konzentration von einer oder mehreren chemischen Spezies im Elektrolyt oder in der gasförmigen Glocke, die die Biokathode umgibt, durchgeführt wird.

11. Verfahren nach Anspruch 2, **dadurch gekennzeichnet, dass** die chemische Spezies Dihydrogen ist.

12. Verfahren nach Anspruch 2, **dadurch gekennzeichnet, dass** die chemische Spezies Methan ist.

13. Verfahren nach Anspruch 10, **dadurch gekennzeichnet, dass** die chemische Spezies ein nicht fermentierbares Molekül ist, wie z. B. eine organische Säure oder ihr Salz, vorzugsweise ausgewählt aus Acetat, Lactat oder Propionat.

14. Verfahren nach einem der vorhergehenden Ansprüche, **dadurch gekennzeichnet, dass** wenn die Bioanode, wie z. B eine Elektrode aus Kohlenstoff, in einen wässrigen Elektrolyt mit einem pH von ungefähr 7 eingetaucht ist, der maximale Wert der Potenzialdifferenz zwischen der Bioanode und einer normalen Wasserstoffelektrode, genannt Referenzelektrode, derart ist, dass das Potenzial der Bioanode kleiner oder gleich 1 V mit Bezug auf die Referenzelektrode, vorzugsweise kleiner oder gleich 0,5 V mit Bezug auf die Referenzelektrode ist, um die Elektrolyse des Wassers zu vermeiden.

15. Elektrochemische Vorrichtung (1) zur Durchführung des Verfahrens nach einem der vorhergehenden Ansprüche, umfassend:
- eine Anode (3) und eine Kathode (6), die in einen Elektrolyt getaucht sind bzw. in einem anodischen Raum (8; 28) und einem kathodischen Raum (9; 29) angeordnet sind, die durch mindestens eine Membran getrennt (4 ;14 ;24) oder miteinander durch eine Salzbrücke verbunden sind, wobei der Elektrolyt (10A) des anodischen Raums Mikroorganismen enthält, ebenso wie der Elektrolyt (10C) des kathodischen Raums, wobei die Elektroden als Bioanode bzw. Biokathode bezeichnet werden,
- eventuell eine Referenzelektrode (7),
- Mittel, die ermöglichen, eine Potenzialdifferenz zwischen der Bioanode und der Biokathode oder zwischen der Bioanode und der Referenzelektrode anzulegen
- wobei die Bioanode und die Biokathode mit einer elektronidchen Vorrichtung zur Steuerung dieser Potenzialdifferenz verbunden sind, **dadurch gekennzeichnet, dass** es Mittel umfasst, die es ermöglichen, die faradaische Leistung der Biokathode zu optimieren, wobei die Mittel Sensoren oder Sonden umfassen, die sich im Elektrolyt (10A, 10C) und/oder in der Gasglocke (12, 15) befinden, die die Bioanode (3) oder die Biokathode (6) umgibt, wobei die Sensoren oder Sonden physisch-chemische Parameter auf der Ebene des anodischen Raums (28) und/oder des kathodischen Raums (39) messen, wie z. B. die Konzentration von einer oder mehreren chemischen Spezies

16. Elektrochemische Vorrichtung nach Anspruch 15, **dadurch gekennzeichnet, dass** die Sensoren (13, 16) oder Sonden (18, 19) mit der elektronischen Vorrichtung (17) verbunden sind.

17. Elektrochemische Vorrichtung nach einem der Ansprüche 15 oder 16, **dadurch gekennzeichnet, dass** der anodische (28) und kathodische (39) Raum durch eine Kationentauschermembran (24) getrennt sind, wobei der anodische Raum einen Eingang (23) und einen Ausgang (25) von Elektrolyt umfasst, die mit Pumpmitteln von Elektrolyt verbunden sind, wobei der kathodische Raum einen Eingang (30) und einen Ausgang (31) von Elektrolyt umfasst, die mit Pumpmitteln verbunden sind, die unabhängig von den Pumpmitteln sind, die mit dem anodischen Raum verbunden sind.

18. Elektrochemische Vorrichtung nach einem der Ansprüche 15 bis 17, **dadurch gekennzeichnet, dass** sie Mittel zur Verschiebung der Bioanode (3) und/oder der Biokathode (6) im Elektorolyten umfasst, die ermöglichen, die eingetauchte Fläche der Bioanode und/oder der Biokathode zu variieren.

## Claims

1. Method of regulating the activity of an electrochemical device (1) comprising an anode (3) and a cathode (6) immersed in an electrolyte that is respectively placed in a separate anode compartment (8) and a cathode compartment (9) that are separated by at least one membrane (4) or connected to one another by a salt bridge, wherein the device optionally comprises a reference electrode (7), and wherein a potential difference is applied between the anode and the cathode, or between the anode and the reference electrode,
**characterized in that** the electrolyte (10A) of the anode compartment as well as the electrolyte (10C) of the cathode compartment each contain microorganisms in suspension or in the form of biofilm(s), wherein the electrodes are respectively referred to as bioanode and biocathode, and wherein the operation of the device is controlled by a double regulation:
- a first regulation, called the priority regulation, of the potential difference between the bioanode and the biocathode or between the bioanode and the reference electrode, between a minimum limit value allowing the development of an electroactive biofilm at the bioanode and a maximum limit value lower than the oxidation potential of said biofilm, and
- a second regulation, called the secondary regulation, set up during the first regulation to optimize the faradic yield of the biocathode.

2. Method according to claim 1,
**characterized in that**, while maintaining the regulation of the potential at the bioanode, the optimization of the activity of the biocathode for the production of particular chemical species is controlled by physico-chemical parameters measured at the cathode compartment (9), such as the concentration of one or more chemical species in the electrolyte or in the gaseous atmosphere surrounding the biocathode, or the rate of gas production at the biocathode.

3. Method according to claim 1 or 2,
**characterized in that**, while maintaining the first potential difference regulation controlling the biological activity at the level of the bioanode, the second regulation controls the optimization of the current density at the biocathode for the production of particular chemical species at the biocathode.

4. Method according to claim 3,
**characterized in that** the optimization of the current density at the biocathode is performed by means of an electronic device to directly fix the current density at the biocathode, and/or through a voltage generator or a potentiostat, allowing variation, preferably with an accuracy of a few millivolts, of the potential of the bioanode, the biocathode and/or the potential difference between the bioanode and the biocathode.

5. Method according to claim 3 or 4,
**characterized in that** the optimization of the current density at the biocathode is achieved by variations in the ratio of the active surface of the bioanode to the active surface of the biocathode.

6. Method according to claim 5,
**characterized in that** the variations in the ratio of the active surface of the bioanode to the active surface of the biocathode are achieved by variations in the immersed surface of the bioanode and/or the biocathode.

7. Method according to claim 6,
**characterized in that** the variations of the immersed surface of the bioanode and/or the biocathode are obtained by variations in the level of the electrolyte in the anode compartment and/or in the cathode compartment.

8. Method according to claim 6 or 7,
**characterized in that** the variations of the active surface of the bioanode and/or the biocathode are obtained by displacements of the bioanode and/or the biocathode in the electrolyte.

9. Method according to any one of claims 5 to 8,
**characterized in that** the variations in the ratio of the active surface of the bioanode to the active surface of the biocathode are achieved by modifying the number of bioanodes in the anode compartment and/or by modifying the number of biocathodes in the cathode compartment.

10. Method according to any one of the preceding claims,
**characterized in that**, while maintaining the regulation of the potential at the bioanode, the optimization of the activity of the biocathode with a view to the production of particular chemical species is carried out by regulation of chemical parameters at the anode compartment, such as the concentration of one or more chemical species in the electrolyte or in the gaseous atmosphere surrounding the bioanode.

11. Method according to claim 2,
**characterized in that** the chemical species is dihydrogen.

12. Method according to claim 2,
**characterized in that** the chemical species is methane.

13. Method according to claim 10,
**characterized in that** the chemical species is a non-fermentable molecule, such as an organic acid or its salt, preferably selected from acetate, lactate or propionate.

14. Method according to any one of the preceding claims,
**characterized in that** when the bioanode, such as a carbon electrode, is immersed in an aqueous electrolyte at a pH of about 7, the maximum value of the potential difference between the bioanode and a normal hydrogen electrode, referred to as the reference electrode, is such that the potential of the bioanode is less than or equal to 1 V relative to said reference electrode, preferably less than 0.5 V relative to said reference electrode, so as to avoid the electrolysis of water.

15. Electrochemical device (1) for carrying out the method according to any one of the preceding claims, comprising:
- an anode (3) and a cathode (6) immersed in an electrolyte respectively placed in an anode compartment (8; 28) and a cathode compartment (9; 29), separated by at least one membrane (4; 14; 24) or connected to one another by a salt bridge, wherein the electrolyte (10A) of the anode compartment as well as the electrolyte (10C) of the cathode compartment each contains microorganisms, wherein the electrodes are respectively called the bioanode and the biocathode,
- optionally a reference electrode (7),
- means for applying a potential difference between the bioanode and the biocathode, or between the bioanode and the reference electrode,
- the bioanode and the biocathode being connected to an electronic device for regulating this difference in potential,
**characterized in that** the device comprises means for optimizing the faradic efficiency of the biocathode, wherein the means comprising sensors or probes arranged in the electrolyte (10A, 10C) and/or in the gaseous atmosphere (12, 15) respectively surrounding the bioanode (3) or the biocathode (6), wherein the said sensors or probes measure physico-chemical parameters at the anode compartment (28) and/or the cathode compartment (39), such as the concentration of one or more chemical species.

16. Electrochemical device according to claim 15,
**characterized in that** the sensors (13,16) or probes (18,19) are connected to the electronic device (17).

17. Electrochemical device according to one of claims 15 or 16, **characterized in that** the anode (28) and cathode (39) compartments are separated by a cation exchange membrane (24), the anode compartment having an electrolyte inlet (23) and an electrolyte outlet (25) connected to electrolyte pumping means, the cathode compartment having an electrolyte inlet (30) and an electrolyte outlet (31) connected to pumping means independent of the pumping means connected to the anode compartment.

18. Electrochemical device according to any one of claims 15 to 17,
**characterized in that** the device comprises means for displacing the bioanode (3) and/or the biocathode (6) in the electrolyte, making it possible to vary the immersed surface of the bioanode and/or the biocathode.
